# EUROPEAN PATENT APPLICATION

(11) **EP 2 537 464 A1**
(43) Date of publication of application: **26.12.2012**
(21) Application number: 11854853.6
(22) Date of filing: 03.08.2011
(51) Int. Cl.: A61B 5/0452

(54) **METHOD AND SYSTEM FOR AUTOMATED DETECTION AND ANALYSIS IN PEDIATRIC ELECTROCARDIOGRAPHY**

(30) Priority: 25.07.2011 CN 201110208781
(71) Applicant: Edan Instruments, Inc., Shenzhen, Guangdong 518000 (CN)
(72) Inventor: LIAO, Yunpeng, Shenzhen Guangdong 518000 (CN); ZHOU, Feng, Shenzhen Guangdong 518000 (CN); LIU, Man, Shenzhen Guangdong 518000 (CN); YANG, Jiaying, Shenzhen Guangdong 518000 (CN); WEI, Daxue, Shenzhen Guangdong 518000 (CN)
(74) Representative: Schmid, Klaus Michael Johannes
(86) International application number: PCT/CN2011/077978
(87) International publication number: WO 2012/092766

(57) **Abstract**

Related to a method and system for automatically detecting and analyzing pediatric ECGs, the invention comprises a signal acquisition system, a lead number determination module, a QRS wave positioning module, a P and T wave positioning module, a template and waveform analysis module, an automatic comparison module and a display and print module. The invention is a computer-aided analysis to the electrocardiograms of pediatric patients under the age of 16, applicable to the electrocardiograms of children acquired by different numbers of leads, and can be more widely used in clinical application. Moreover, the invention adopts the combined single-lead and multi-lead method to position the easily interfered characteristic points of P, QRS and T waves of pediatric ECGs. Therefore, this method can avoid the errors caused by single-lead calculation, guarantee the accuracy of parameter calculation, and consequently guarantee the accuracy of final automatic analysis results.

## Description

### Field of the invention

The invention relates to an electrocardiogram automatic analysis system, especially a method and system for automatically detecting and analyzing the electrocardiograms of children under the age of **16**.

### Background of the invention

Dutch physiologist W. Einthoven, the father of electrocardiogram, invented electrocardiogram (ECG) in 1895, which is taken as intermediate processed data and later widely applied to clinical analysis. Today, 100 years later, ECG has been indispensable intermediate processing data in clinical application and in particular in the very common heart disease analysis. Now, the 12-lead ECG used in clinics, can synchronously acquire, amplify and record electrocardiosignals.

With the progress of the ECG research work based on computer analysis, the function of computers in ECG analysis has been generally recognized, and the number of ECGs analyzed by the computers is also increased yearly. Major electrocardiographs (ECG) in the current market basically have the function of automatic analysis.

Most ECGs automatically analyzed by computers are for adults and seldom for pediatrics. Taken the analysis standards of adult ECGs as reference, the available ECG automatic analysis system seldom considers the characteristic points of pediatric ECGs. Therefore, the accuracy of automatic analysis is very low, and thus inconvenience is brought to doctors in clinical analysis.

Considering the inherent characteristics of pediatric ECGs, four lead placement means for acquisition of pediatric ECGs in clinics are generally adopted as follows: 1. standard 12 leads; 2. 9 leads (three chest leads among the standard 12 leads are not used); 3. 6 leads (only limb leads among the standard 12 leads are used. 6 chest leads are not used); and 4. 15 leads (extra three right chest leads *(V_{3R}, V_{4R}, V_{5R})* are used in the standard 12 leads). The available ECG automatic analysis system generally analyzes pediatric ECGs by the standard 12-lead means and seldom considers the actual clinical application.

Difference is obviously found between pediatric and adult ECGs, the younger the greater. The differences are represented by the heart rate and the waveform, amplitude and time of various intervals and various leads, and characteristics of pediatric ECGs can be summarized as follows: 1. heart rates are faster; 2. the time of various intervals and various waves is shorter; 3. the amplitude of various waves, particularly the amplitude of precordial leads, is higher; 4. the right ventricle is dominant and the electrical axis is deviated to the right; and 5. T waves are certainly changed at different age periods. Taken the analysis standards of adult ECGs as reference, the available ECG automatic analysis system seldom considers the characteristic points of pediatric ECGs. Therefore, the accuracy of automatic analysis is very low, and thus valid references cannot be provided for clinicians.

### Summary of the invention

To solve the problems in analyzing pediatric ECGs through the current electrocardiogram automatic analysis system, the invention provides a method and system for automatically detecting and analyzing the electrocardiograms of pediatric patients under the age of 16 with high accuracy and high stability.

To solve the problems, the invention comprises a signal acquisition module, an analysis module and an output module, wherein the signal acquisition module is used for acquiring ECG signals; the analysis module is used for analyzing ECG data and generating analytic data; the output module is used for outputting the analytic data; the signal acquisition module comprises lead groups; the analysis module comprises a lead number determination module, a QRS wave positioning module, a P and T wave positioning module, a template and waveform analysis module and an automatic comparison module which are connected with each other in turn; the lead number determination module is connected with the signal acquisition module; the automatic comparison module is connected with a display and print module; the QRS wave positioning module comprises a single-lead QRS wave recognition unit and a multi-lead QRS wave combined positioning module which are connected in series between the lead number determination module and the P and T wave positioning module; the P and T wave positioning module comprises a single-lead P and T wave recognition unit and a multi-lead P and T wave combined positioning module which is connected in series between the QRS wave positioning module and the template and waveform analysis module; the template and waveform analysis module comprises an average template selection unit, a unit for positioning the characteristic points of a QRS wave of the average template and identifying the waveform pattern of the QRS wave, and a unit for positioning the characteristic points of P and T waves of the average template and identifying the polarity of the P and T waves connected with each other in turn; and the average template selection unit is connected with the P and T wave positioning module; and the unit for positioning the characteristic points of the P and T waves and identifying the polarity of the P and T waves connected with the automatic comparison module.

The automatic detection and analysis method of the invention comprises the following steps: a) signal acquisition: connecting leads in one lead group by a signal acquisition device and acquiring signals of various leads in the lead group which adopts 12 leads or 15 leads, and step a) comprises the following substeps: determining whether the number of leads of the lead group is 12 leads or 15 leads by the signal acquisition device according to the signal conditions of various leads in the lead group; and further determining whether the number of valid leads is 6, 9, 12 or 15. b) parameter selection and calculation: firstly, determining the number of the valid leads in the lead group, selecting the required leads subsequent calculation and analysis according to the lead number, but relevant parameters of fallen leads are not calculated; secondly, positioning the valid leads one by one and performing the multi-lead combined positioning on QRS waves, which comprises the following substeps: searching for the QRS waves of the valid leads one by one, positioning and screening out the acquired QRS waves by multi-lead means; thirdly, positioning the valid leads one by one and performing multi-lead combined positioning on P and T waves of each beat according to the obtained QRS wave parameters, so as to obtain P and T wave parameters of each beat; Meanwhile, selecting an average template for each valid lead according to the obtained QRS wave parameters. The process of creating the average template for each valid lead is as follows: 1. Creating templates according to the waveform pattern of QRS waves acquired by various leads; 2. Determining dominant beats, and average processing the dominant beats subjected to signal to generate the average template; 3. Calculating the characteristic points and waveform pattern of a QRS wave and the characteristic points and polarity of P and T waves of each average template, in which the specific means is as follows: 1. identifying the QRS waveform pattern and setting a local boundary; 2. determining the positions and forms of the characteristic points of the P or T wave according to the maximum and minimum extremes in a QRS wave window; and 3, comprehensively comparing all the valid leads, scaling the boundaries of various subwaves, and calculating parameters such as PR interval, QT interval, QRS wave duration and P wave duration, etc. c) The process of signal analysis: comparing the acquired characteristic points and waveform of the QRS wave and the acquired characteristic points and polarity of the P and T waves with default characteristic parameters and parameters in a criterion library, so as to obtain comparison results and output parameters and comparison results of the QRS, P and T waves.. In the process of creating the default characteristic parameters and the criterion library, the reference values are divided into male and female in view of gender or 7 intervals with 14 values in view of age, namely Birth-1 month, 1-6 months, 7-12 months, 1-3 years, 3-8 years, 8-12 years and 12-16 years. The default characteristic parameters and the criterion library can be set in the process of implementing the above detection and analysis steps and can also be set in advance.

For better implementation of the automatic analysis on pediatric ECGs, the concept of the invention is as follows: firstly, automatically recognizing pediatric ECGs acquired by different lead acquisition systems; secondly, calculating the characteristic parameters of electrocardiosignals of various leads; thirdly, creating an average template for each lead electrocardiogram and calculating the characteristic parameters of the average template; fourthly, presetting a criterion library for pediatric ECGs; and fifthly, analyzing pediatric ECGs by computers according to the characteristic parameters and the criterion library, so as to realize the automatic analysis on pediatric ECG.

The advantages of the invention are: 1. The invention performs computer-aided analysis on complicated and changeable electrocardiograms of pediatric patients under the age of 16, so it can effectively reduce the work amount of manual analysis for clinicians; 2. The invention is applicable to pediatric ECGs acquired by different numbers of leads, and it is more applicable to clinical application; 3. The invention automatically compares the waveforms and calculation parameters of the electrocardiograms of pediatric patients of different age periods and different genders with the automatic analysis of the preset criterion library, and it can effectively provide references for the clinicians; and 4. The invention adopts the single-lead and multi-lead combined method to position of easily interfered characteristic points of P, QRS and T waves of the electrocardiograms of children, and thus it can avoid the errors caused by single-lead calculation and guarantee the accuracy of parameter calculation, and consequently guarantee the accuracy of final automatic analysis results.

### Brief description of the drawings

Fig. 1 is a structure diagram of the invention;

Fig. 2 is an overall flowchart the invention;

Fig. 3 is a lead number recognition flowchart;

Fig. 4 is a flowchart illustrating the single-calculation-lead detection of QRS waves for pediatric ECGs;

Fig. 5 is a flowchart illustrating the single-calculation-lead detection of P and T waves for pediatric ECGs; and

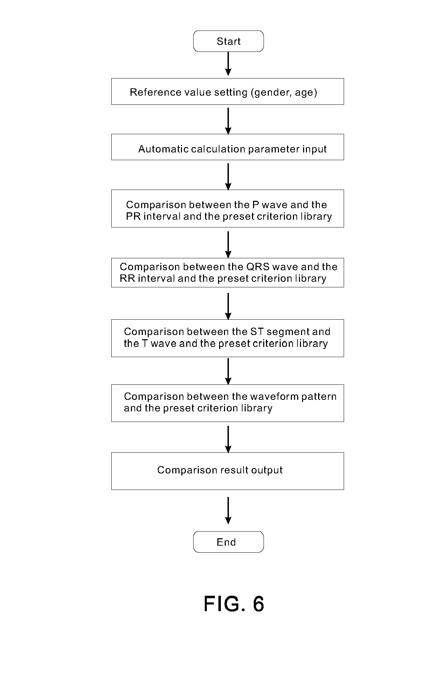

Fig. 6 is a flowchart illustrating the criterion automatic comparison and analysis of pediatric ECGs.

### Detailed description of the preferred embodiments

Further detailed description is given to the invention with the attached drawings 1 to 6 and the preferred embodiments.

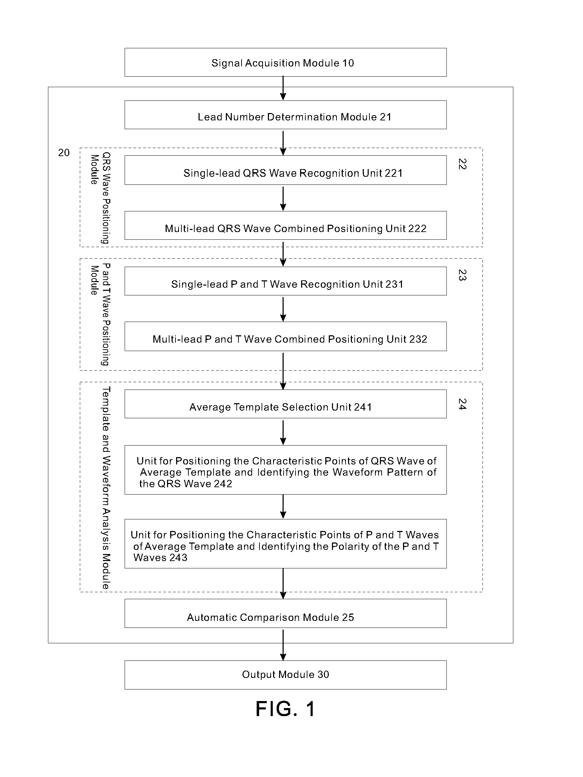

Signals processed by the invention are pediatric ECGs acquired by a signal acquisition device. The invention mainly performs signal processing and recognition on acquired data of pediatric ECGs, so as to complete the automatic analysis. Fig. 1 is a structure diagram of the invention. As illustrated in the text, the system comprises a signal acquisition module 10 for acquiring ECG signals, an analysis module 20 for analyzing ECG data and generating analytic results, and an output module 30 for outputting the analytic results, wherein the signal acquisition module 10 comprises a signal acquisition lead group 11; the analysis module 20 comprises a lead number determination module 21, a QRS wave positioning module 22, a P and T wave positioning module 23, a template and waveform analysis module 24 and an automatic comparison module 25 which are connected with each other in turn; the lead number determination module 21 is connected with the signal acquisition module 10; the automatic comparison module 25 is connected with the output module 30; the QRS wave positioning module 22 comprises a single-lead QRS wave recognition unit 221 and a multi-lead QRS wave combined positioning unit 222 which are connected in series between the lead number determination module 21 and the P and T wave positioning module 23; the P and T wave positioning module 23 comprises a single-lead P and T wave recognition unit 231 and a multi-lead P and T wave combined positioning unit 232 which are connected in series between the QRS wave positioning module 22 and the template and waveform analysis module 24; the template and waveform analysis module 24 comprises an average template selection unit 241, a unit 242 for positioning the characteristic points of a QRS wave of an average template and identifying the waveform pattern of the QRS wave, and a unit 243 for positioning the characteristic points of P and T waves of the average template and identifying the polarity of the P and T waves, which are connected with each other in turn; the average template selection unit 241 is connected with the P and T wave positioning module 23; and the unit 243 for positioning the characteristic points of the P and T waves and identifying the polarity of the P and T waves is connected with the automatic comparison module 25.

The processing flow of the whole system is as follows:

The signal acquisition module 10 is responsible for the acquiring and preprocessing (referring to hardware filter processing) pediatric ECGs, In view of actual clinical demands, height and body weight are less for younger pediatric patients (particularly under the age of 6), so the placement of 6 chest electrodes on the chest is very difficult. As for younger pediatric patients, usually only three chest electrodes (V1, V3 and V5 are generally selected) are placed on the chest in clinical practice for acquiring ECG signals of chest leads. And valid signals acquired then are 9-lead data (6 limb leads and 3 chest leads). Sometimes, only the electrocardiogram rhythm is considered as for pediatric ECGs. Herein, the clinician usually only places limb electrodes and does not place the chest leads. And valid signals acquired then are 6-lead data (6 limb leads). As for other fallen leads, acquired signals are invalid and the signal value is 0, the values of them are exclusive in the subsequent calculation and analysis. As for older pediatric patients, usually 12-lead electrocardiograms are adopted, and the acquired valid signals are standard 12-lead data (6 limb leads and 6 chest leads). The acquisition mode of the above pediatric ECGs is standard 12-lead acquisition mode, and the number of leads for the three modes is different. Due to the obvious differences between pediatric and adult ECGs, right ventricles of the pediatric patients are dominant compared with those of the adults. Therefore, for the complete observation of pediatric ECGs, particularly the pediatric patients with suspected heart disease, the clinician selects 15-lead electrocardiogram (standard 12 leads and three right chest leads *(V_{3R}, V_{4R}, V_{5R})),* and the acquired valid signals are 15-lead data. The above is the fourth acquisition mode of pediatric ECGs, namely 15-lead mode.

The lead number determination module 21 determines the above four acquisition modes and obtains valid leads for calculation.

The QRS, P and T wave positioning modules comprise a single-lead QRS wave recognition unit, a multi-lead QRS wave combined positioning unit, a single-lead P and T wave recognition unit and a multi-lead P and T wave combined positioning unit. The single-lead QRS recognition unit is used for completing the detection of a QRS wave of a single calculation lead. The acquired QRS wave of each calculation lead is inputted into the multi-lead QRS wave combined positioning unit, and QRS waves at the same position are determined by all the calculation leads, so as to recognize correct QRS waves. P (T) waves of each calculation lead are detected by the single-lead P and T wave recognition unit according to the position of the recognized QRS wave, so as to obtain candidate points of the P (T) waves. In addition, the candidate points are recognized by the multi-lead P and T wave combined positioning unit, so as to determine the positions and number of the P and T waves of each beat.

The template and waveform analysis module comprises the average template selection unit, which comprise a unit for positioning the characteristic points of the QRS wave of the average template, a unit for identifying the waveform pattern of the QRS wave, and a unit for positioning the characteristic points and identifying the polarity of the P and T waves of the average template. The average template selection unit is to search for dominant beats on each calculation lead by utilization of the information of each beat acquired by the QRS, P and T wave positioning modules, and perform signal averaging processing on the dominant beats to generate the average template. Therefore, an average template will be generated for each calculation lead. The average template represents the waveform information of all the dominant beats of each calculation lead, and the waveform information of the dominant beats can be reflected by the measurement and analysis of the average template. After the selection of the average template, the measurement information such as the type of the QRS wave of the average template and the onset and offset of the QRS wave group is recognized by the unit for positioning the characteristic points and identifying the waveform pattern of the QRS wave. In addition, the measurement information such as the onset and offset and the polarity of the P and T waves of the average template is recognized by the unit for positioning the characteristic points and identifying the polarity of the P and T waves according to the QRS wave information.

The automatic comparison module is responsible for completing the comparison with a preset comparison criterion library and completing criterion output. The comparison criterion library is preset according to reference values (age and gender). The automatic comparison module is used for completing the waveform comparison of pediatric ECGs according to the beat information acquired by calculation and outputting comparison results.

The display and print module is used for displaying and printing data of pediatric ECGs and the electrocardiogram automatic measurement and comparison results.

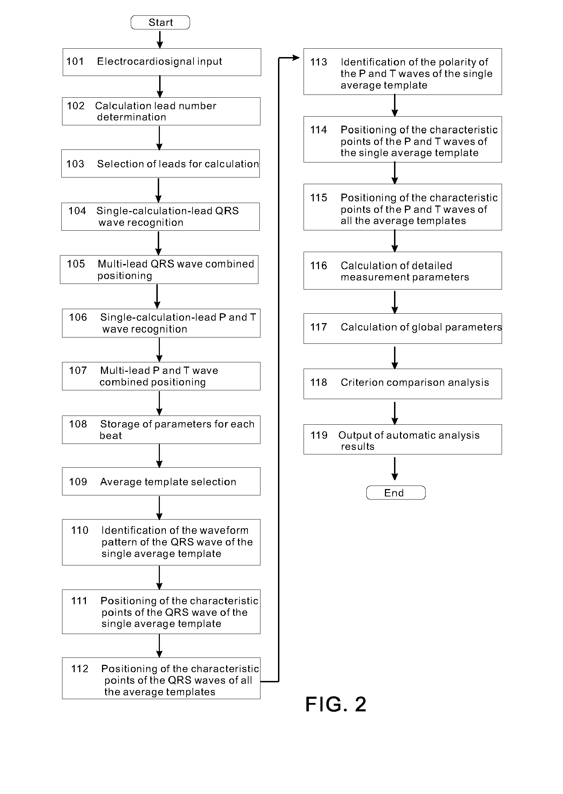

The main implementation of the invention is the automatic measurement and diagnosis of pediatric ECGs. The main processing flow of the method for automatically detecting and analyzing pediatric ECGs, provided by the invention, is shown as illustrated in Fig. 2. The method mainly comprises the following steps:

101. Inputting ECG signals, data of pediatric ECGs and corresponding gender and age.

102. Performing lead number determination on the ECG signals acquired in step 101, and determining whether the ECG signals are 6-lead data, 9-lead data, 12-lead data or 15-lead data.

103. Selecting leads for calculation according to the lead number obtained in step 102, in which fallen leads are abandoned and only leads with actual acquisition signals are selected.

104. Performing single-calculation-lead QRS wave recognition on the electrocardiogram data acquired in step 101 according to the number of calculation leads obtained in step 103.

105. Performing multi-lead QRS wave combined positioning on the electrocardiogram data acquired in step 101 according to the QRS wave information acquired in step 104 and the number of the calculation leads obtained in step 103.

106. Performing single-calculation-lead P and T wave recognition on the electrocardiogram data acquired in step 101 according to the QRS wave information acquired in step 105 and the number of the calculation leads acquired in step 103.

107. Performing multi-lead P and T wave combined positioning on the electrocardiogram data acquired in step 101 according to the P and T wave information acquired in step 106 and the number of the calculation leads acquired in step 103.

108. Storing P/QRS/T wave parameters of each beat calculated in steps 104 to 107.

109. Selecting an average template for the electrocardiogram data acquired in step 101 according to the QRS wave parameters obtained in step 105, in which one average template is selected for each calculation lead according to the number of the calculation leads obtained in step 103.

110. Performing QRS waveform pattern recognition on the single average template obtained in step 109, in which the QRS waveform pattern is identified for the average template of each calculation lead and distinguished into QRS, R, RS, QS and other types.

111. Positioning the characteristic points of the QRS wave of the single average template obtained in step 109, in which the onset and offset of the QRS wave of each average template and the onset and offset of various subwaves (for example, Q wave, R wave, S wave) of the QRS wave are positioned according to the information calculated in step 110.

112. Positioning the characteristic points of the QRS waves of all the average templates obtained in step 109, in which unified onset and offset of the QRS waves are positioned for the average templates of all the calculation leads according to the onset and offset of each average template, calculated in the step 111; the first onset of the QRS waves in all the average templates is taken as the unified onset of the QRS waves of the average templates; and the final offset of the QRS waves in all the average templates is taken as the unified offset of the QRS waves of the average templates.

113. Performing P and T wave polarity recognition on the single average template obtained in step 109, in which the polarity of the P and T waves is recognized for the average template of each calculation lead according to the information calculated in steps 110 and 111 and distinguished into positive, negative, biphasic and flat,; and the number of the P waves can be numerous or zero.

114. Positioning the characteristic points of the P and T waves of the single average template obtained in step 109, in which the onset and offset of the P and T waves of each average template are positioned according to the information calculated in steps 110, 111 and 113.

115. Positioning the characteristic points of the P and T waves of all the average templates obtained in step 109, in which unified onset and offset of the P(T) waves are positioned for the average templates of all the calculation leads according to the onset and offset of the P(T) waves of each average template, calculated in step 114; the first onset of the P(T) waves in all the average templates are taken as the unified onset of the P(T) waves of the average templates; and the final offset of the P(T) waves in all the average templates are taken as the unified offset of the P(T) waves of the average templates.

116. Solving for detailed measurement parameters of each average template according to the information calculated in steps 110, 111, 113 and 114, including P-wave duration, PR interval, QRS-wave duration, Q-wave duration, R-wave duration, S-wave duration T-wave duration, QT interval, QRS wave type, STj amplitude and other parameters.

117. Solving for global parameters of the electrocardiogram data according to the information calculated in steps 112 and 115, including heart rate, P-wave duration, PR interval, QRS -wave duration QT interval, QTc interval, electrical axis of heart for P/QRS/T waves, RV5 (the amplitude of R wave for V5 lead), SV1 (the amplitude of S wave for V1 lead), RV6 (the amplitude of R wave for V6 lead), SV2 (the amplitude of S wave for V2 lead) and other parameters.

118. Performing waveform and parameter comparison on pediatric ECGs according to the default reference values (age and gender) and the characteristic parameters (including P/QRS/T wave parameters of the average template of each beat, detailed measuring parameters and global parameters) obtained in the above steps and the preset criterion library, so as to obtain comparison results.

119. Outputting automatic analysis results, in which the characteristic parameters of pediatric ECGs, automatically measured in the above steps, and the comparison results are outputted.

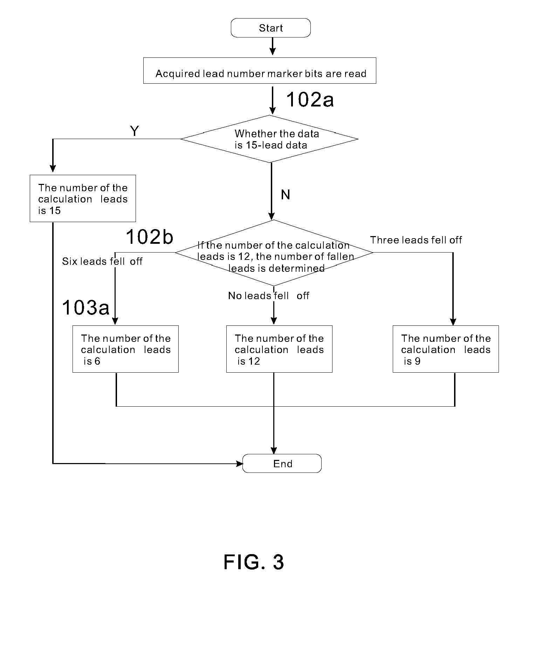

The flows of steps 102 and 103 are shown as illustrated in Fig. 3:

102a. Reading lead marker bits in the acquired data according to the signal conditions of various leads in an acquisition lead group, and determining whether the data is 12-lead data or 15-lead data, in which if the data is 15-lead data, the acquisition mode is 15-lead mode and the number of the calculation leads is 15.

102b. Reading lead-off marks in the acquired 12-lead data according to the signal conditions of various leads in the acquisition lead group. If the number of the leads is 12, in which if 6 leads are fallen off, the acquisition mode is 6-lead mode and the number of the calculation leads is 6; if 3 leads are fallen off, the acquisition mode is 9-lead mode and the number of the calculation leads is 9; and if no lead fell off, the acquisition mode is 12-lead mode and the number of the calculation leads is 12.

103a. Not calculating relevant parameters of the fallen leads.

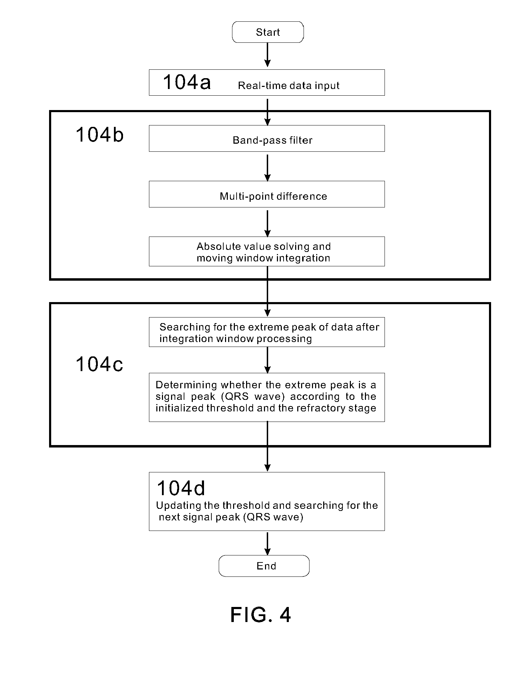

The main process of step 104 is shown as illustrated in Fig. 4:

104a. Inputting data of the single calculation lead;

104b. Preprocessing the data of the calculation lead, including band-pass filter, multi-point difference, absolute value solving and moving window integration. After the data preprocessing, the characteristics of the QRS wave are greatly highlighted; most noise is inhibited; and the detection rate of the QRS wave is improved.

104c. Searching for an extreme peak (noise peak or signal peak) of the data after the integration window processing; and adopting the extreme peak for threshold estimation, and taking a signal peak exceeding the threshold and within a refractory period as the QRS wave, in which the heart rate detection range of children is between 15 and 350 bpm in view of large change of the heart rate of the electrocardiograms of children, while the heart rate detection range of adults is usually between 30 and 300 bpm; and the refractory period for the detection of the QRS wave is adjusted according to the heart rate detection range.

104d. Updating the threshold and searching for the next signal peak (QRS wave) until all the QRS waves are found.

Step 105 also comprises the following steps:

105a. Performing position correction on the QRS wave detected by each calculation lead, in which as for QRS waves at the same position, if more than one third of the calculation leads detect the existence of the QRS waves, for example, 4 leads among 9 calculation leads detect the existence of the waves, the existence of the QRS waves is recognized; or else, it will be regarded as interference.

105b. Detecting the onset and offset of the QRS wave of each calculation lead by the slope threshold method after the QRS wave is determined in the above step.

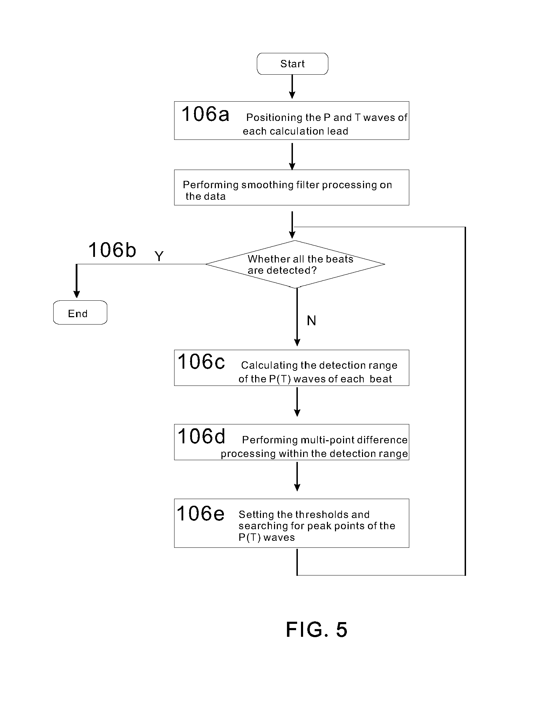

The main process of the step 106 is shown as illustrated in Fig. 5:

106a. Positioning the P and T waves of each calculation lead according to the QRS wave position determined in steps 104 and 105.

106b. Performing smoothing filter processing on the data inputted and determining whether all the beats are detected, in which if so, the detection is over; if not, the next step 106c is executed.

106c. Calculating the search ranges (i.e. search windows) of the P(T) waves of each beat, in which the P wave search window is the previous period of time of the QRS wave and the T wave search window is the later period of time of the QRS wave; and the search window of each beat is related to each RR interval.

106d. Performing multi-point difference processing on the data within the detection range, so as to improve the peak characteristics and reduce the noise.

106e. Setting thresholds according to peak points in the search windows, and taking those exceeding the thresholds as candidate points of the P(T) waves.

Step 107 also comprises the following steps:

107a. The candidate points obtained in step F are subjected to multi-lead combined judgment, in which if more than one third of the calculation leads detect the existence of the waves, the existence of the waves is recognized; or else, it will be regarded as interference.

107b. The processing means of the P and T waves is different, the T wave of each beat is unique but the number of the P waves may be zero or one or numerous, and the candidate points are distinguished in accordance with the principle during the judgment.

Step 109 also comprises the following steps:

109a. Selecting the average template for each calculation lead, in which firstly, the forms of the beats (QRS waves) acquired by various leads are classified (template creation), so as to determine dominant beats; and secondly, the dominant beats are subjected to signal averaging processing to generate the average template.

The reference values mentioned in step 118 are set as below:

118a. According to the characteristics of pediatric ECGs, the reference values of the preset criterion library are divided into male and female in view of gender or 7 intervals with 14 reference values in view of age, namely birth-1 month, 1-6 months, 7-12 months, 1-3 years, 3-8 years, 8-12 years and 12-16 years.

The above is only further detailed description given to the invention with the attached preferred embodiments. It should not be considered that the preferred embodiments of the invention are only limited to the description. It should be understood by those skilled in the art that various simple deductions or replacements can also be made without departing from the concept of the invention and should be all within the scope of protection of the invention.

## Claims

1. A method for automatically detecting and analyzing pediatric ECGs, comprising: a) signal acquisition: connecting leads in a lead group and acquiring signals of various leads in the lead group by a signal acquisition device; b) parameter selection and calculation: firstly, determining the number of valid leads in the lead group; secondly, positioning the valid leads one by one and performing multi-lead combined positioning on QRS waves; thirdly, positioning the valid leads one by one and performing multi-lead combined positioning on P and T waves of each beat according to QRS wave parameters obtained, so as to obtain P and T wave parameters of each beat; fourthly, meanwhile, selecting an average template for each valid lead according to the QRS wave parameters obtained; fifthly, calculating the characteristic points and waveform pattern of a QRS wave and the characteristic points and polarity of P and T waves of each average template; and c) signal analysis: comparing the acquired characteristic points and waveform pattern of the QRS wave and the acquired characteristic points and polarity of the P and T weaves with default characteristic parameters and parameters in a criterion library, so as to obtain comparison results; and outputting parameters of the QRS, P and T waves and the comparison results.

2. The method for automatically detecting and analyzing pediatric ECGs according to claim 1, wherein in the step a), the lead group adopts 12 leads or 15 leads; and the step a) comprises the following substeps: a1) determining whether the number of leads of the lead group is 12 or 15 by the signal acquisition device according to the signal conditions of various leads in the lead group; and a2) further determining whether the number of the valid leads is 6, 9, 12 or 15.

3. The method for automatically detecting and analyzing pediatric ECGs according to claim 2, wherein the step b), comprises the following substeps: b1) selecting the required leads for subsequent calculation and analysis according to the lead number, but not calculating relevant parameters of fallen leads are not calculated; b2) searching for QRS waves of the valid leads one by one; and b3) positioning and screening out the recognized QRS waves in the step b2) by multi-lead means.

4. The method for automatically detecting and analyzing pediatric ECGs according to claim 2 or 3, wherein the step b), also comprises the following substeps: b4) creating the average template for each valid lead, in which firstly, templates are created according to the waveform patterns of QRS waves acquired by various leads; secondly, dominant beats are determined; and thirdly, the dominant beats are subjected to signal averaging processing to generate the average template.

5. The method for automatically detecting and analyzing pediatric ECGs according to claim 4, wherein in the step b), the process of calculating the characteristic points and waveform pattern of the QRS wave and the characteristic points and polarity of the P and T waves of each average template, comprises the following substeps: b5) firstly, identifying the QRS waveform pattern and setting a local boundary; b6) secondly, determining the positions and forms of the characteristic points of the P or T wave according to the maximum and minimum extremes in a QRS wave window; and b7) thirdly, comprehensively comparing all the valid leads, scaling the boundaries of various subwaves, and calculating parameters such as PR interval, QT interval, QRS wave duration and P wave duration.

6. The method for automatically detecting and analyzing pediatric ECGs according to any one of claims 1 to 3, wherein in the process of creating the default characteristic parameters and the criterion library in the step c), the reference values are divided into male and female in view of gender or 7 intervals with 14 reference values in view of age, namely birth-1 month, 1-6 months, 7-12 months, 1-3 years, 3-8 years, 8-12 years and 12-16 years.

7. A system for automatically detecting and analyzing pediatric ECGs, comprising a signal acquisition module, an analysis module and an output module which are connected with each other in turn, wherein the signal acquisition module used for acquiring ECG signals; the analysis module used for analyzing ECG data and generating analytical data; the output module used for outputting the analytical data; the signal acquisition module comprising a signal acquisition lead group; the analysis module comprising a lead number determination module, a QRS wave positioning module, a P and T wave positioning module, a template and waveform analysis module and an automatic comparison module which are connected with each other in turn; the lead number determination module connected with the signal acquisition module; and the automatic comparison module connected with the output module.

8. The system according to claim 7, wherein the QRS wave positioning module comprises a single-lead QRS wave recognition unit and a multi-lead QRS wave combined positioning unit which are connected in series between the lead number determination module and the P and T wave positioning module.

9. The system according to claim 7, wherein the P and T wave positioning module comprises a single-lead P and T wave recognition unit and a multi-lead P and T wave combined positioning unit which are connected in series between the QRS wave positioning module and the template and waveform analysis module.

10. The system according to any one of claims 7 to 9, wherein the template and waveform analysis module comprises an average template selection unit, a unit for positioning the characteristic points of a QRS wave of an average template and identifying the waveform pattern of the QRS wave, and a unit for positioning the characteristic points of P and T waves of the average template and identifying the polarity of the P and T waves, which are connected with each other in turn; and the average template selection unit is connected with the P and T wave positioning module; and the unit for positioning the characteristic points of the P and T waves and identifying the polarity of the P and T waves is connected with the automatic comparison module.
